# EUROPEAN PATENT SPECIFICATION

(11) **EP 2 116 202 B1**
(45) Date of publication and mention of the grant of the patent: **26.10.2011**
(21) Application number: 07715817.8
(22) Date of filing: 16.02.2007
(51) Int. Cl.: A61B 17/42

(54) **Uterine manipulator for complete removal of human uteri**
Gebärmutter-manipulator zur vollständigen entfernung der menschlichen Gebärmutter
Dispositif de manipulation utérin permettant de procéder à l'extirpation de l'utérus chez les humains

(43) Date of publication of application: 11.11.2009
(73) Proprietor: Lopez Zepeda, Marco Antonio, Jalisco 44620 (MX)
(72) Inventor: Lopez Zepeda, Marco Antonio, Jalisco 44620 (MX)
(74) Representative: ABG Patentes, S.L.
(86) International application number: PCT/MX2007/000014
(87) International publication number: WO 2008/100125

(56) References cited:
- EP-A1- 0 615 727
- WO-A2-03/015643
- DE-A1- 10 341 561
- US-A- 5 643 285
- US-A- 5 645 561
- US-A1- 2003 187 334
- US-A1- 2006 241 652

## Description

### BACKGROUND OF THE INVENTION

### Field of Invention

The present invention relates to devices for application in surgical procedures in the medical field, and more specifically in the area of gynecology, as it is a device that is used for total removal (hysterectomy) of human uteri.

### Related Prior Art.

The most feared complication when performing a hysterectomy (removal of uterus) by any route of approach are the urinary tract lesions including bladder, and especially the ureters. Laparoscopic hysterectomy in the incidence of urinary tract injury is 0.02 - 1.7%, going mostly in securing (tie) the uterine vessels, as the zone to tie the uterine artery is located near the junction with the ureter and the bladder.

Total laparoscopic hysterectomy (HTL) is a real challenge for laparoscopic surgeons because the surgery is technically very difficult and demanding, and often is accompanied by prolonged surgical time, therefore the incidence of complications is greater.

The use in the HTL of devices such as the uterine manipulator to remove the complete human uterus facilitates the realization of it, reducing the time and the incidence of surgical complications.

There are currently several tools to remove the uterus by laparoscopy in humans, surgical procedure in which the patient's abdomen is expanded through the introduction of an innocuous gas, usually carbon dioxide, to facilitate the surgery. One such tool is the Mc Cartney tube consisting of a cylindrical, slightly curved, which at one end has a lid with a valve whose function is to prevent gas from leaking, and at the opposite end has a rounded smooth edge, whose role is to expose the endopelvic fascia. However, this device has the disadvantage that it is simply a silastic tube in which one of its edges serves to demarcate the edge of the fascia to carry out the colpotomy (incision) and to prevent leakage of pneumoperitoneum, but said device has no fixation and mobilization system of the uterus, so that it does not facilitates the procedure because it fails to provide an optimal view of the vulnerable structures, which disadvantage is more evident in large uteri.

Another known device being currently used for the same purpose is the Wattiez manipulator(Clermont-Ferrand) consisting of: a long cylindrical tube, which carries at one end an inverted L-shaped, rotatable tongue having rounded smooth edges, adapted to expose the endopelvic fascia at the opposite end of the tube, near to the terminal portion is a short, rectangular sleeve-shaped device, whose function is to provide rotary movements to the tube and thus to the tab located on, the other end. Inside said tube, a cylindrical rod is housed, which at the end where the tube tongue is located has a threaded portion to which externally threaded conical tips are adapted to be fixed to the cervical canal; a rectangular handle is located at the opposite end of the cylindrical rod, which is adapted to move the inner rod. In the middle of the outer tube, a circular and conical sliding device comprising three soft rings is disposed to occlude the vagina and prevent escape of gas. However, this uterine manipulator has the disadvantage that is very large and difficult to maneuver, and for the exposure of the fascia it has a rotary latch instead of a complete cap, so the exposure and protection of structures such as the bladder and uterine vessels may be insecure and not uniform.

Another instrument of this type is known as RUMI system that is a uterine injector manipulator consisting of two main components: 1.- a reusable handle and 2.- a single use, disposable sterile tip that adheres to said handle. The handle consists of a grip element, a rod, a clamping drum and a blocking trigger. The handle has a "L" shaped design and 20 cm. (8") long along the rod and 10 cm. (4") long along the grip element. The drum (placed at the extreme position of said rod) is used to position the tip and rotate it into an arc of 140 degrees. The grip element of manipulator is 90 degrees relative to the rod. By turning the grip element, while the blocking trigger is pressed, allows the tip positioning drum to rotate and thus to change the position of the tip. When released, the blocking trigger secures the position of rotation of the grip element and the clamping drum. The single use tip and associated body is manufactured in silicone USP Class IV medical grade, an internal wire for support and a balloon fastened from outside at the distal end of the tip, which is also manufactured in silicone USP Class IV. When properly inflated, the balloon positions the tip in the uterus. The tips have three delivery ports along the arrow thereof. One port is for inflating the balloon with sterile saline. The remaining two ports near the distal end position of the tip are for administrating contrast media. Two silicone catheters extended from the tip serve as balloon extensions and for delivering the dye into the cavity. The balloon catheter, 30 cm. long (12 inches), has a white syringe connection, a hose clamp and a white luer connector. The clamp is operated manually to open or close the closure seal of the globe. The catheter of the contrast medium is clear in color and 60 cm. (24") long and it has a white luer connector to adjust the syringe of the dye. To accommodate the different uterine sizes, the tips are provided in different diameters and lengths of the arrows. Another variant of this device is known as pneumo-occluder system consisting in the RUMI system adapted to include a cap in the proximal end, which press fits to delimit the endopelvic fascia and an inflatable silicone ring being placed at the middle portion of the instrument inside the vagina so as to occlude this latter and prevent leakage of gas. It is an instrument primarily designed for mobilizing the uterus, and has very fragile elements so its use to remove large uterus is very difficult.

The Hohl's uterine manipulator is another of the tools currently used in surgical procedures. This manipulator consists in a non-hollow, long cylindrical device adapted to include at one end a conical-shaped threaded cone to fix in the cervical canal, said device has in turn an extensor coupled to the tip thereof. This cylindrical device slides within a T-shaped hollow cylindrical tube, which includes a cap fastened to the end opposite to the "T" by means of an external threaded portion and nut connection. At the T-shaped end, a screw is provided inside the "T" to fix the internal cylindrical rod. This manipulator has the disadvantage that it is completely straight and lacks of articulation in the cap area so that no anteflexion and 90 degree movements are available, hence in some difficult patients and in certain technical difficulty circumstances, the access to the rear of the fascia can be very difficult or even dangerous.

US 2003/187334 A1 describes a device to aid in the manipulation of the uterus and vaginal vault, said device being in T-arrangement (18) on the proximal end to allow rotation of the device for insertion and also the translation of the cap (19) along the lug (12). This T-bar is not separable from the lug once the device is in position, and subsequent manipulation of the uterus is done manually in-situ using the T-bar.

DE 10341561 A1 discloses a uterine manipulator comprising a gun type motion or mobilization device in accordance with the preamble of claim 1. This manipulator (1) has a position lock (25) and a base element (3) with a sliding side bar (5). A further grip (29) slides under tension between a holding position and a release position.

WO 03/015643 A2 discloses a manipulating device suitable for manipulation of an uterus in colpotomy, hysterectomy or the like procedures in accordance with the preamble of claim 1, the device including: a guide means for insertion into a cervix; a locating means for locating the guide means in the cervix; an engaging means for engaging an internal wall of the cervix; and an operating system for controlling movement of the guide means inside the cervix.

In comparison with other similar instruments, the new manipulator has the following advantages:
1) Compared with Mc Cartney tube, the new one fixes and mobilizes the uterus in a safe manner, especially large uterus.
2) Compared with the Wattiez manipulator of Clermont Ferrand, the new one has a complete cap and it is easier to maneuver due to its size.
3) Compared with the RUMI system, the new one is stronger and therefore facilitates the total laparoscopic hysterectomy even in very large uterus.
4) Compared with the Hohl uterine manipulator, the new one has an articulated gun having anteversoflexion and lateral movement to improve the access angle to the posterior endopelvic fascia and uterine vessels.

### SUMMARY OF THE INVENTION

The advantages of the invention against conventional uteri manipulation devices materialize through a novel device designed for complete removal of human uteri by laparoscopy, which includes a system for fixing the uterus and uterine cervix and mobilizing them to allow an adequate exposure of all anatomic structures, and providing an optimal viewing angle of the vulnerable anatomical structures as the ureters and bladder, said new device also including a system for exposure of the fascia and seating of the vagina when making the incision of the same, thereby allowing the surgical procedure to continue in a more secure manner and preventing the leakage of the pneumoperitoneum. The manipulator of this invention is characterized by comprising the following main components: 1) a gun type motion device having an ergonomic handle which serves as a grip of said device, a first shaft, which is fixed by one end thereof to said handle, extending from this latter in an essentially perpendicular direction, a second sliding shaft being essentially parallel to the first arrow, a grip next to the handle, which is fastened to the second shaft to move jointly therewith, wherein the handle has two confronting extensions between which the first shaft extends and in which the operator may insert the fingers to retract said grip towards the handle, and a securing drum or cylinder to which the distal ends of the first and second shafts are connected and being adapted to move pivotally from a horizontal position to a second position to reach a rotary movement upwards by at least 90°, 2) an uterus fixation system comprising a lug releasely coupled to said securing cylinder, said lug having a threaded section including sharp edges, the diameter of which decreases forwards to be fixed to the cervix and ends at its front end into a threaded tip, an extension member having a longitudinal internally threaded conduct for coupling to the tip of said lug, said extension member extending into the uterine fundus, and a cap which is coupled to the lug to assist this latter in securing and manipulating the cervix and to prevent pneumoperitoneum leakage, and 3) an applicator assembly for positioning properly the lug and the cap on the cervix, which is a bar having at one end connecting means for releasable coupling to the lug and on the other end an element for facilitating the screwing of the lug on the cervix, and a sliding tube that being inserted on the bar, behind the cap, to slide uniformly this latter along a longitudinal slot in the bar to a position in which it engages with the lug to secure the cervix.

### BRIEF DESCRIPTION OF THE FIGURES

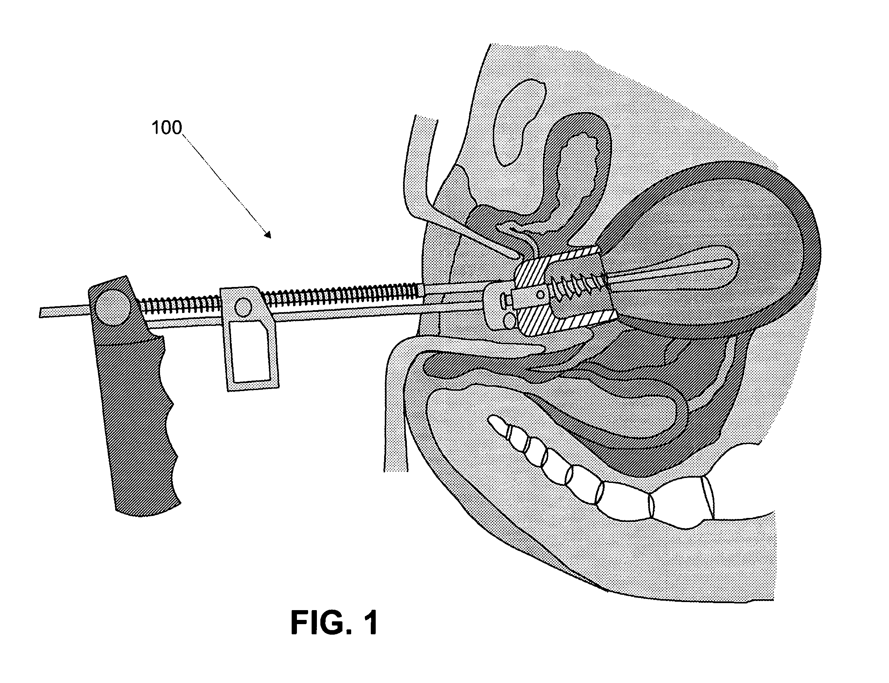

Figure 1 is a schematic side sectional view of the manipulator in use to remove the uterus in a patient whose anatomy is shown in cut away to allow seeing the placement of the manipulator represented in a first operating position.

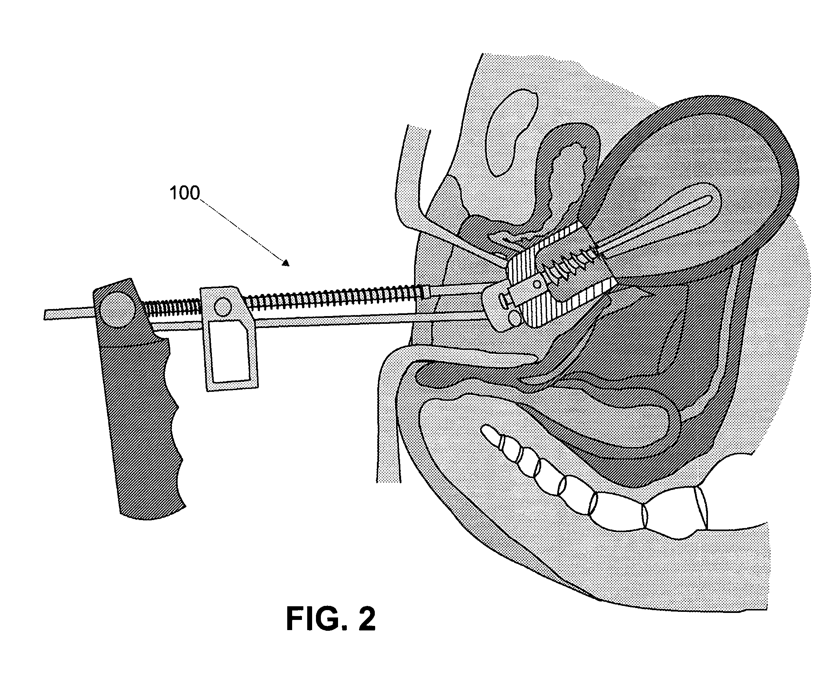

Figure 2 is another schematic side view similar to the preceding one except that the manipulator is shown in a second anteroflexion position to get a better angle of access to the posterior endopelvic fascia and uterine vessels.
Figures 3A and 3B are side views of the gun type motion device shown in its two operating positions, the first one with the completely horizontal securing cylinder and the second one with the same cylinder rotated in an upward movement as a result of a anteversoflexion movement.

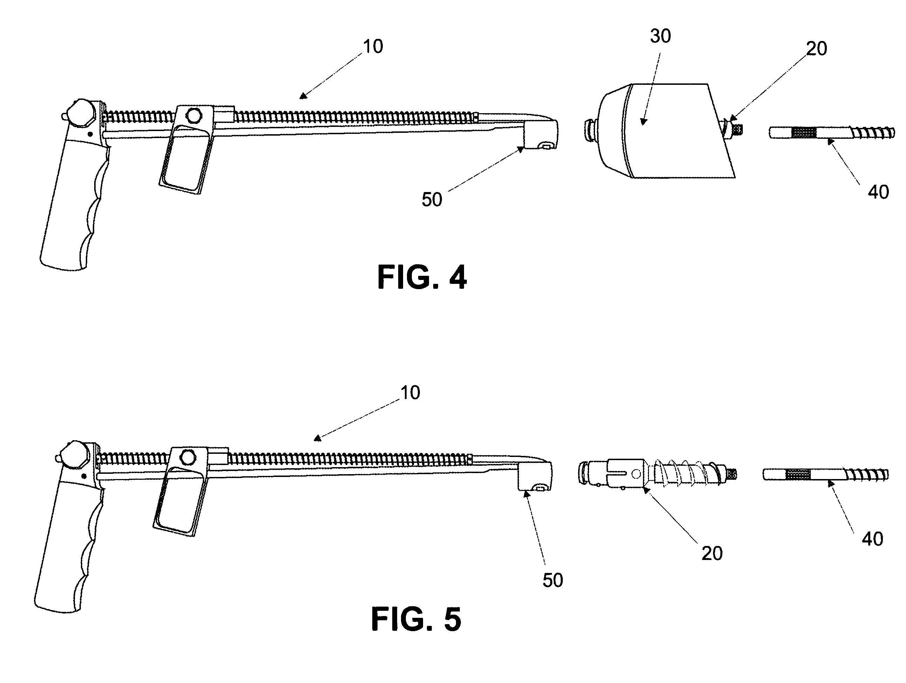

Figure 4 is a side view of the complete uterine manipulator shown in disassembled condition.
Figure 5 is a side view similar to preceding one except that this has omitted the cap to allow seeing the threaded lug.
Figure 6 is a side view of the assembly used for applying the lug and the extension member, wherein these two elements are shown in disassembled condition.
Figure 7 is a view similar to that of Figure 6 with the difference that in this one the cap is placed in position for coupling to the lug and the pusher tube used to slide the cap until the shown position.
Figure 7A is an enlarged view of the front end of the rod of the applicator assembly.
Figures 8A, 8B and 8C are perspective views of the securing cylinder or drum represented in a lug securing condition (Fig. 8A), lug releasing condition (Fig. 8B) and a rear view of the latter (Fig. 8C).
Figures 9A and 9B are side views of the threaded lug, wherein the second view shows the type of securing mechanism that interacts with that of the cap for coupling together.
Figures 10A, 10B and 10C are side views -the first two- of the elements that fix and move the uterus, shown in assembled and disassembled condition, respectively; and the last one (Fig. 10C) is a perspective front view of the cap.
Figures 11A and 11B are side views of two types of extensions, one a blunt point extension and the other a sharp point extension.
Figure 12 is an exploded view of the securing assembly used to fix the sliding rod in a determined position.
Figure 12A is a perspective bottom view of the slotted screw of the fixing assembly of the previous figure.

### DETAILED DESCRIPTION OF THE PREFERRED EMBODIMENT OF THE INVENTION

The new uterine manipulator (100) for total removal of human uteri is an instrument designed based on experience gained by the use of different manipulators and knowledge of the advantages and shortcomings of each of them. From these latter, very specially, a uterine manipulator has been designed which meets certain basic requirements such as: a system for fixing the uterus and cervix to allow an adequate exposure of all anatomic structures to provide an optimal angle of attack of vulnerable anatomical structures such as ureters and the bladder, and a system for exposure of the fascia and sealing of the vagina when this is incised, which allows to continue the procedure with an adequate exposure and preventing the escape of pneumoperitoneum.

This is achieved by applying a conical-shaped lug (20) which is to be threaded to the cervical canal to fix the cervix, the free end of said lug is previously attached by threading to a tip (40)that reaches the uterine fundus to allow an adequate mobilization of the uterus to be removed. At least three different sizes are designed both for the lug (20) and the extension (40) in order to be implemented depending on the size of the cervix and uterine cavity of the patient. Moreover, two types of tips (40)are designed, ones having blunt point (42) and others having sharp point (44), these latter being suitable for use in the event that there exist tumors in the uterine cavity so that the sharp point extension (44) passes through the tumoral tissue without deviating the desired trajectory toward the uterine fundus in order that the exposure and mobilization of the uterus is always uniform on both sides. The lug and extension assembly (20, 40) is placed in the cervical canal with an applicator (60), which will be described hereafter, designed specifically for this purpose. Next, a Teflon cap (30) is placed, also available in at least three different sizes, with the shorter front edge and the longer rear edge, said cap being slid by means of a pusher tube (66) along the applicator (60) of lugs (20). The applicator has a guide groove along which the cap slides until the latter is perfectly positioned in engagement with the system for applying and fixing the lug. Then, the applicator is removed and the free end of the lug is coupled to the fixing cylinder of the motion gun. By following the above procedure, the proper fastening of the uterus to the manipulator is achieved, thereby allowing an optimum performance of this device to take advantage of all its advantages, such as: proper exposure at the front of all the anatomical structures such as round ligaments, salpinges, utero-ovarian ligaments and broad ligaments. In addition, when pushing the motion device through the vaginal, the bladder is folded back from the incision area to remain on behind incision zone of the fascia and therefore there is no need to dissect or move said bladder, thereby avoiding denervation thereof.

By means of the articulated anteflexion movement of up to 90 degrees of the uterine manipulator, the back of the uterus is fully exposed which permits to work safely away from the descending colon and rectum. Also, the utero-sacral ligaments are taken care because the incision area of the fascia is located above the insertion of the utero-sacral ligaments; thus, the Frankenhauser nervous plexus is preserved, gaining from 2 to 3 cm. of the length of the vagina because the utero-sacral ligaments are not cut. Moreover, in pushing the manipulator along the vagina is avoided the risk of a ureteral injury, which is the junction of the ureters to the uterine artery, because they are moved away up to a distance of 4 to 5 cm., thereby reducing significantly the risk of ureteral injury.

It is important to point out that of all the above advantages in the exposure are achieved despite part of the fascia has been cut, and there is no leakage of pneumoperitoneum due to the design of the caps that allow the cutting of the fascia without losing exposure. In summary, the uterine manipulator to remove complete uteri exposes all the human anatomical structures especially at vulnerable areas such as ureters and bladder, and also avoids motion that may cause bladder denervation, moves away ureters from the danger zone to a distance of about 4 to 5 cm., thereby decreasing the risk of injury, and moves away the descending colon and rectum due to their anteflexion movement.

With reference to the attached figures, the uterine manipulator (100) mainly comprises a gun-shaped mobilizer or motion device (10), a system for fixation and mobilization (20, 30, 40) of the cervix and uterus, and an applicator assembly (60) useful to place in position the elements of the fixation system that come into contact with the organ to be removed.

The device (10) has a first non-sliding round shaft (11), which is fixed by one end to an ergonomic handle (12), the distal end of which is pivotally connected to a securing cylinder or drum (50), a second sliding shaft substantially parallel to the first arrow and being disposed over this latter, and whose distal end is also pivotally connected to the securing cylinder (50) at a point farther than that of the distal end of the first shaft (11), a retractable grip (15) disposed next to the handle (12) to be held by the index and middle fingers of the user and retracted towards the handle to produce retro anteversoflexion and retroversoflexion movements in the cylinder (50), the grip (15) defines a through passageway between two confronting extensions and is fixed to the second shaft (14) by a hole and screw mechanism to move jointly with said arrow. To this end shaft (14) is inserted through a passage in the grip (15) and springs (17a, 17b) surrounding the sliding shaft (14) to make this and the associated mobile elements, securing cylinder (50) and grip (15) to recover its original position. A first spring (17a) abuts on one end against the front portion of the grip and on the other end against a prominence in the shaft (14) towards the distal end thereof while the other spring (17b) makes contact on one end against the top of the handle and on the other end against the rear portion of the grip, said spring (17b) is adapted to restore the initial straight position of the uterine manipulator.

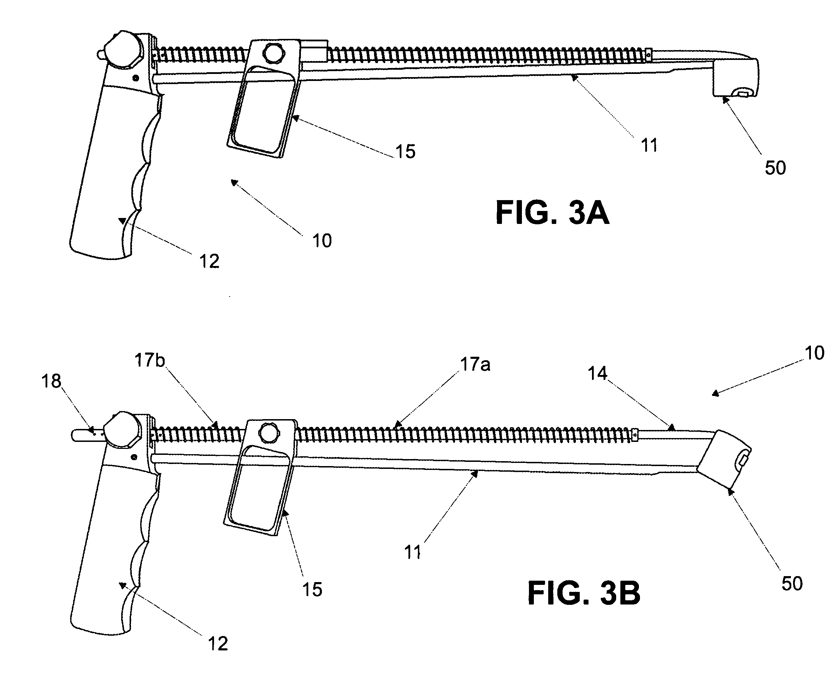

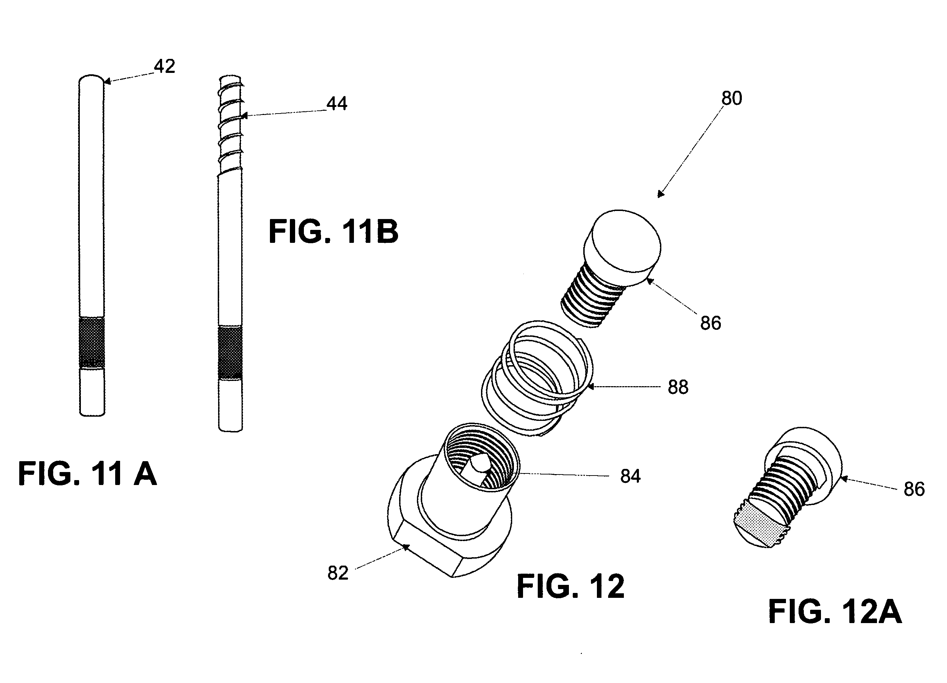

To secure the position of anteversoflexion of securing cylinder (50) and associated elements (20, 30, 40) upon actuating the grip (15) to slide the mobile shaft (14), the device (10) has a mechanism (80) of the type of hollow nut (82) (Figure 12) which is internally threaded and has a center tipped bolt (84) which penetrates the perforations (18) of the sliding shaft (14). Nut (82) is screwed with a complementary slotted screw (86) externally threaded (Fig. 12A), thereby allowing the shaft (14) to slide therethrough. When threading the screw (86) and the nut (82), bolt (84) introduces into one of the holes (18) of the slidingarrow (4) for keeping thus the instrument at a determined fixed position (Figures 3A, 3B). A spring (88) disposed between the hollow nut (82) and the slotted screw (86) provides the tension required for proper operation.

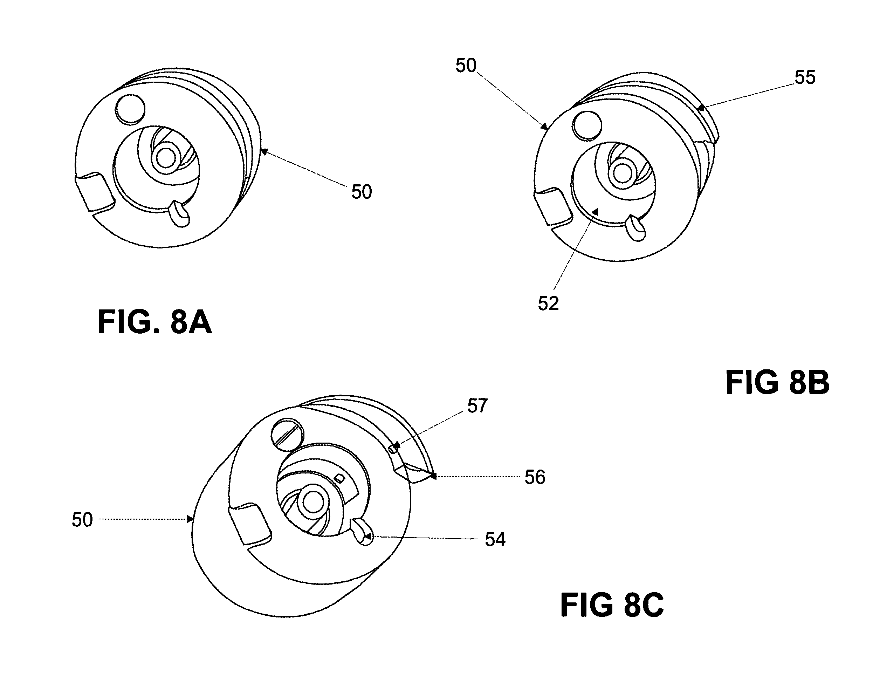

The securing cylinder or drum (50) (Figures 8A to 8C) has a hole (52) at its front part, in which a section (28) of a lug (20) is inserted for fastening to the cylinder. In addition, the cylinder (50) has a small groove (54) on its inner wall to receive therein the rear bolt (23) of the threaded lug. Another important element in the cylinder (50) is a securing and releasing mechanism of lug (20) which comprises a small semicircular arm (55) embodied to the cylinder periphery and pivotally connected on one end thereof inside the cylinder. The other opposite end of said arm (55) has lateral mobility to actuate the securing and releasing mechanism (20) so as to release the lug from coupling with the cylinder when moving outwardly the free end (56) of arm. This semicircular arm (55) has wedge (57) which when in securing position is inserted into the circumferential recess (24) of a lug being housed in the hole (52) to hold it and keep it in position.

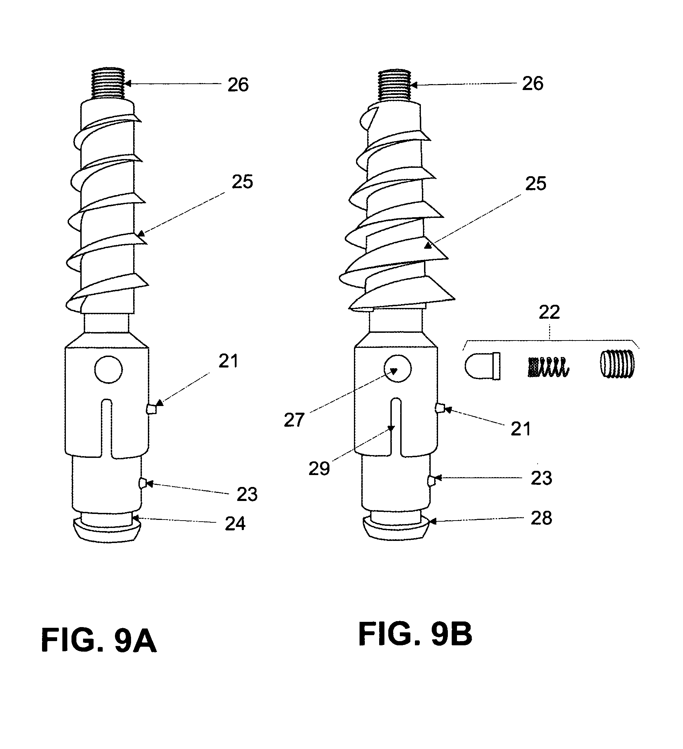

Each lug (20) comprises a body with a main threaded section (25), which is to be fixed to the cervix, a tip (26) threaded at its forward end for engagement with an extension member (40), which is the element that is inserted to the fundus of the uterus to properly fix this organ during the surgical procedure. Approximately half of the lug body (20) is conical shaped with sharp threads (25), immediately behind which a through hole (27) is located in which a securing mechanism (22) of the type of ball spring adjustment (Figure 9B) is housed, this mechanism interacting with complementary coupling elements in the cap (30) to fasten this latter to the lug during the surgical procedure. The lug/cap combination, once fixed to the cervix, will constitute the seal that prevents leakage of the pneumoperitoneum.

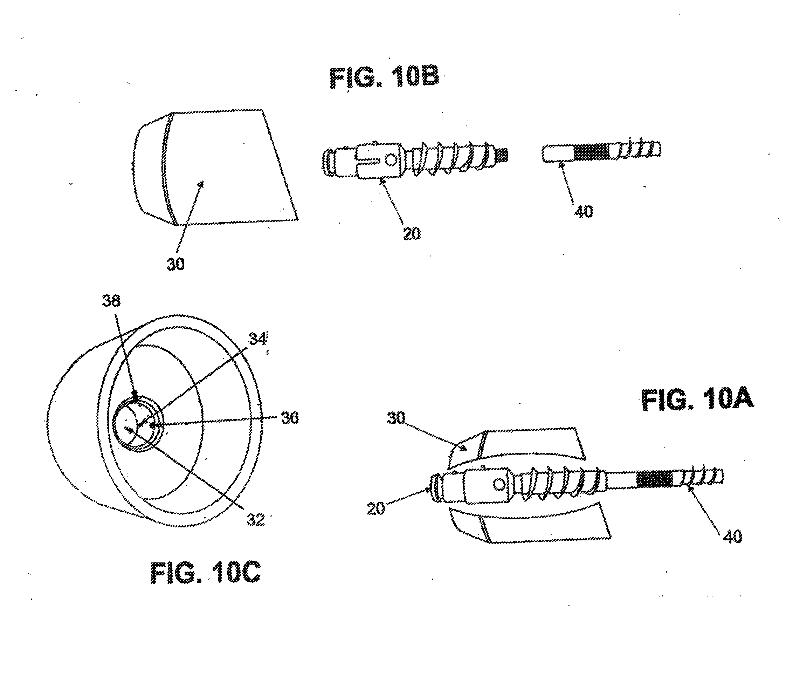

With reference to Figure 10C, the lug has a slot (29) to guide the cap (30) to coupling with the same. Also, lug has a neck section (28) useful to enter into the securing cylinder (50). The circumferential recess (24) is disposed in the neck section in which the semicircular arm wedge (57) of cylinder (50) is inserted so as to fasten the threaded lug (20) to the cylinder (50).

Another feature of each lug (20) consists in side bolts (21, 23), wherein bolt (21) is to be coupled with the internal lateral slot (38) of cap (50) and the other bolt (23) temporarily engagement to a slotted section (69) in the rod (62) of the applicator assembly (60) during the procedure followed to place the lug in the cervix, and subsequently with the lateral groove of the securing cylinder (50), once coupled with it.

Due to the anatomical diversity of patients, it has been conceived that the manipulator has at least three elements of each of the following components: threaded lugs (20), extensions (40) of each type, that is, blunt point extensions (42) or sharp point extension (44), and caps (30).

Each extension (40) has, in the end facing to the lug (20), an internally threaded conduct (not shown) to ensure there the corresponding lug. At the other end, the extension will be blunt point (42) or sharp point (44) ended. These latter are to be used when there are tumors inside the uterus cavity so that they can be penetrated to ensure that the adequate fixing of the organ to be removed.

Each cap (30) is designed in such a manner that its front edge is shorter than the rear edge and also rounded at its distal end. In addition, the cap (30) has a central through hole (32) through which the applicator bar (60) is inserted when the cap will be coupled with the lug (20). The cap has a bolt (34) interacting with a longitudinal guide slot in the applicator bar (64) to slide during the placement thereof. The bolt (34), together with a small depression (36) in alignment therewith, interact with slot (29) and the ball/spring securing mechanism of lug (20) to maintain the cap in position and prevent the cap retraction.

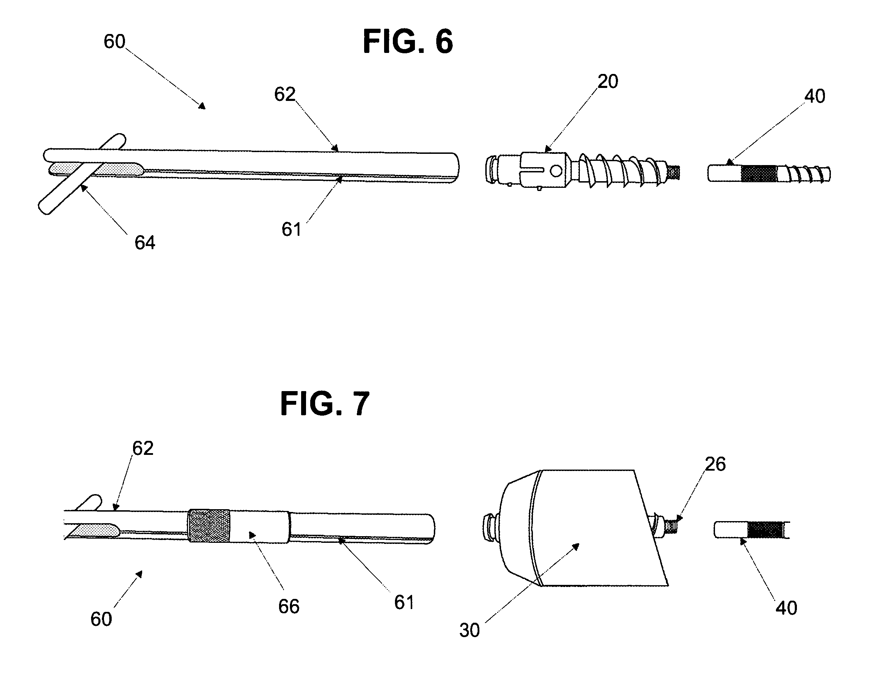

The applicator assembly 60 (Figs. 6 and 7) is a complementary element of the uterine manipulator which serves to position and fix the cervix and uterus of the patient. The assembly (60) comprises a central bolt (62) consisting in a cylindrical bar having at one end a transverse bar (64) that serves as a handle to facilitate the fixation of lug in the cervix. The other end of the bar has a cavity at the tip to receive the neck of lug (20), while a L-shaped semicircular groove (69) is disposed at the outer wall of the bar to receive therein the posterior bolt (23) of the threaded lug during the process of placing of said lug on the cervix. Further, the bar has a longitudinal slot (61) therealong which serves as a sliding guide during the process of coupling of the cap (30) with the lug. Finally, another element of the applicator (60) is a pusher tube (66) which is inserted to slide on the bar (62) and serves to slide smoothly the cap (30) into the vagina and cervix during the positioning of the elements (20, 30) that prevent the leakage of pneumoperitoneum.

### Bibliography:

1.- Mc Cartney, tube, Obstet., Gynecol 1995 85: 293-296
2.- Heinberg Eric MD, MPH, Crawford Benjamin L. III MD, Total laparoscopic hysterectomy in Obese versus Non-obese Women, Obstetrics and Gynecology, Volume 103(4), April 2004, pp 674-680 3.- Koh Ch., Pneumo Ocluder J Amer. Assoc. Gynecological Endoscopy, May 1998, Vol 5, No. 2
4 .- Hohl M K, Badener manipulator Frauenheilkunde Aktuell 8/2/99; 5-11

Although the invention has been described in the context of the preferred embodiment, it will be evident for a skill in the field that the scope of the concept exemplified hereinbefore extends beyond the specifically disclosed and illustrated model to other possible alternative embodiments of the invention that are feasible or viable. Furthermore, although the invention has been described in detail, any expert in the field to which the invention belongs may deduce that several of the constitutive elements of the manipulator may be replaced or others incorporated in the light of the foregoing description without altering essentially the result for which it was designed.

In view of the above, it will be understood that various elements of the device can be combined with or replaced by others to conform alternate modes of materialization leading to the same result. Therefore, it is intended that the scope of the present invention is not construed as limited to the particular forms described, but rather it is determined by the contents of the following claims.

## Claims

1. A kit of parts for the manipulation of the uterus (100) for complete removal of human uteri to be used in laparoscopic surgical procedures, comprising a system for fixing the uterus and cervix and mobilized to allow adequate exposure of all anatomic structures, providing an optimal viewing angle of the anatomical structures which are vulnerable as the ureters and bladder, and a system for exposure of the fascia and sealing of the vagina to make the cut of that to continue with the surgical procedure in a way more secure and preventing the leakage of the pneumoperitoneum; said kit (100) comprising:
a) a gun type motion or mobilization device (10) comprising an ergonomic handle (12) acting as an instrument grip, a first shaft (11) fixed by one end to the handle and extending in a direction essentially perpendicular to the handle, a second sliding shaft (14) essentially parallel to the first shaft, a grip (15) next to the handle, which is fastened to the second shaft (14) to move jointly with the latter, wherein the grip (15) has two confronting extensions between which the first shaft extends and in which fingers of the operator are introduced to retract the grip (15) towards the handle (12), and a securing cylinder (50) to which the distal end of the first and second shafts are connected, said securing cylinder (50) being adapted to move pivotally from a horizontal operating position to a second operating position in which it can reach a rotational movement upwards of at least 90°;
b) an uterus fixation system (20, 30, 40) comprising a lug (20) releasably coupled to said securing cylinder (50), said lug (20) having a threaded section including sharp edges, the diameter of which decreases forwards to be fixed to the cervix and ends at its front end into a threaded tip (26), an extension member (40) having a longitudinal internally threaded conduct for coupling to the tip (26) of said lug (20), said extension member (40) extending into the uterine fundus, and a cap (30) which is coupled to the lug (20) to assist this latter in securing and manipulating the cervix and to prevent pneumoperitoneum leakage; said kit being **characterized in** further comprising:
c) an applicator assembly (60) for positioning properly the lug (20) and the cap (30) on the cervix, which comprises a bar having at one end connecting means for releasable coupling to the lug (20) and on the other end an element (64) for facilitating the screwing of the lug (20) on the cervix, and a sliding tube (66) that is inserted over the bar (62), behind the cap (30), to slide uniformly this latter along a longitudinal slot (61) in the bar to a position in which it engages with the lug to secure the cervix.

2. The kit of claim 1, wherein said securing cylinder (50) further includes means (57) to retain and release the lug.

3. The kit of claim 1, wherein said lug (20) further includes means (24, 28) for fastening same to the securing cylinder.

4. The kit of claim 1, wherein said lug (20) further includes means (21) for coupling with said cap (30).

5. The kit of claim 1, wherein said cap (30) includes means (38) for coupling with the lug (20) and for sliding along the applicator bar (62).

6. The kit of claim 1, wherein the connection of the distal end of the first and second shafts (11, 14) of the gun device (10) to the securing cylinder (50) is a pivotal connection in both cases, thereby said securing cylinder can rotate upwards in a determined range.

7. The kit of claim 1, wherein said means to retain and release said lug (20) from said securing cylinder (50) consist in a bolt and ball mechanism (22) which inserts into slots (27) and a spring-loaded wedge device (54, 55, 56, 57).

8. The kit of claim 1, wherein the connection of the distal end of the second shaft (14) of the gun device (10) with the securing cylinder (50) remains in a point further on the connection of the first shaft (11) in order that the cylinder can rotate upward when the grip is retracted.

9. The kit of claim 1, wherein the mobilization device (10) includes spring means (17a, 17b) surrounding the sliding shaft (14) to produce the traction and retraction the movement of said shaft (14) from one to another operating positions.

10. The kit of claim 1, wherein the extension member (40) has a sharp tip (44) for use if required to go through tumors in the uterus.

11. The kit of claim 1, wherein the mobilization device (10) includes means (80) to keep the uterus fixation system in a determined fixed position.

## Patentansprüche

1. Ein Satz Teile für die Behandlung der Gebärmutter (100) zwecks vollständiger Entfernung der menschlichen Gebärmutter, um in chirurgischen Bauchspiegelungsprozessen benutzt zu werden, welches ein System für die Festlegung und Bewegung der Gebärmutter und des Mutterhalses aufweist um eine geeignete Freilegung aller anatomischen Strukturen zu erlauben, indem es einen optimalen Sichtwinkel auf die anfälligen anatomischen Strukturen die verletzt werden können, wie zum Beispiel die Harnleiter und die Blase erlaubt, und ein System für die Exposition der Faszie und Versiegelung der Scheide beim schneiden aufweist um die chirurgische Behandlumg mit grösserer Sicherheit durchführen zu können und ein Luftleck im Bauchfell zu vermeiden; wobei dieser Satz (100) folgendes aufweist:
a) eine Bewegungsvorrichtung (10) nach Art einer Pistole mit einem ergonomischen Griff (12) der als Greifinstrument dient, einen ersten mit einem Ende am Griff fixierten Schaft (11) der sich im wesentlichen senkrecht vom Griff erstreckt, und einen zweiten, gleitenden Schaft (14) der sich im wesentlichen parallel zum ersten Schaft erstreckt, einen am Griff nahestehenden Haltering (15) der am zweiten Schaft (14) befestigt ist um sich zusammen mit diesem zu bewegen, wobei der Haltering (15) zwei gegenüberstehende Verlängerungen aufweist zwischen denen sich der erste Schaft erstreckt und in denen die Bedienungsperson die Finger einsteckt um disen Haltering (15) in Richtung Griff (12) zurückzuziehen, und einen Haltezylinder oder Haltetrommel (50) an welchem das distale Ende des ersten und zweiten Schafts angeschlossen wird, wobei dieser Zylinder (50) so ausgelegt ist, dass er sich um einen Drehzapfen drehend von einer waagerechten Arbeitsposition in eine zweite Arbeitsposition bewegt wobei er eine drehende Bewegung um mindestens 90° nach oben ausführen kann,
b) ein Gebärmutterfixiersystem (20,30,40) mit einem Bolzen (20) der sich trennbar an den Haltezylinder (50) anschliesst, wobei dieser Bolzen (20) einen Gewindeabschnitt mit scharfen Kanten aufweist dessen Durchmesser nach vorne abnimmt um an den Mutterhals fixiert zu werden und an seinem vorderen Ende mit einer Gewindespitze (26) endet; eine Erweiterung (40) umfassend einen mit Innengewinde versehenen sich längs erstreckenden Kanal, Erweiterung die an die Spitze (26) des Bolzen (20) angeschlossen wird, wobei sich diese Erweiterung (40) bis zum Gebärmuttergrund erstreckt; und eine Haube (30) welche sich an den Bolzen (20) anschliesst um ihm beim halten und behandeln des Mutterhalses behilflich zu sein und einen Luftleck am Bauchfell zu vermeiden; wobei dieser Satz **dadurch gekennzeichnet ist, dass** er ausserdem
c) eine Ansatzeinheit (60) um den Bolzen (20) und die Haube (30) richtig im Mutterhals einzusetzen aufweist, wobei diese Einheit einen Stab der an einem Ende mittel um trennbar mit dem Bolzen (20) verbunden zu werden aufweist und am anderen Ende ein element (64) um das einschrauben des bolzens (20) in den Mutterhals zu vereinfachen, und ein gleitendes Rohr (66) aufweist welches hinter der Haube (30) auf den Stab (62) aufgeschoben wird um diese längs einer im Stab vorgesehenen sich längs erstreckenden Führungsnute bis an eine Stelle gleiten zu lassen in der sie in den Bolzen einhakt um den Mitterhals zu fixieren.

2. Satz gemáss Anspruch 1, **dadurch gekennzeichnet dass** der Haltezylinder (50) Haltemittel (57) aufweist um den Bolzen zu halten und ihn freizugeben.

3. Satz gemäss Anspruch 1, **dadurch gekennzeichnet dass** der Bolzen (20) ausserdem Mittel (24, 28) aufweist um am Haltezylinder gehalten zu werden.

4. Satz gemäss Anspruch 1, **dadurch gekennzeichnet dass** der Bolzen (20) ausserdem Mittel (21) aufweist um an die Haube (30) angeschlossen zu werden.

5. Satz gemäss Anspruch 1, **dadurch gekennzeichnet dass** die Haube (30) Mittel (38) aufweist um an den Bolzen (20) angeschlossen zu werden und um längs dem Stab (62) der Ansatzeinheit zu gleiten.

6. Satz gemäss Anspruch 1, **dadurch gekennzeichnet dass** der Anschluss der Pistolen (10) Vorrichtung an den Haltezylinder (50) über dem distalen Ende des ersten und zweiten Schafts (11, 14) in beiden Fällen ein Drehanschluss ist, wodurch sich dieser Zylinder um einen feststellbaren Bereich nach oben drehen lässt.

7. Satz gemäss Anspruch 1, **dadurch gekennzeichnet dass** die Mittel um den Bolzen (20) im Haltezylinder (50) zu halten und vom Haltezylinder (50) zu befreien eine in Nuten (27) eingesetzte Bolzen und Kugel Vorrichtung (22) und eine federbetriebene (54, 55, 56, 57) Keilvorrichtung sind.

8. Satz gemäss Anspruch 1, **dadurch gekennzeichnet dass** das distale Ende des zweiten Schafts (14) der Pistolen (10) Vorrichtung an einem Punkt am Haltezylinder (50) angeschlossen wird der weiter vorne liegt als derjenige des ersten Schafts (11), damit der Zylinder sich nach oben drehen kann wenn der Haltering zurückgezogen wird.

9. Satz gemäss Anspruch 1, **dadurch gekennzeichnet dass** die Bewegungsvorrichtung (10) den gleitenden Schaft (14) umgebende Federn (17a, 17b) umfasst, um die Zug- und einziehbewegung des Schafts (14) von der einen und der anderen Betriebslage bewerkstelligen zu können

10. Satz gemäss Anspruch 1, **dadurch gekennzeichnet dass** die Erweiterung (40) eine Vorschneidespitze (44) aufweist, welche benutzt wird falls Geschwülste in der Gebärmutter durchdrungen werden müssen.

11. Satz gemäss Anspruch 1, **dadurch gekennzeichnet, dass** die Bewegungsvorrichtung (10) Mittel (80) umfasst um das System für die Festlegung der Gebärmutter in einer vorgegebenen Stellung zu halten.

## Revendications

1. Un kit d'instruments pour la manipulation de l'utérus (100) pour l'extirpation complète d'utérus humains, qui s'utilise dans des procédures chirurgicales par laparoscopie, qui comprend un système pour fixer l'utérus et le col de l'utérus et les mobiliser afin d'avoir une exposition adéquate de toutes les structures anatomiques, facilitant ainsi un angle de vision optimale des structures anatomiques vulnérables telles que les uretères et la vessie, et un système d'exposition du fascia et de scellage du vagin au moment de couper à l'intérieur de celui-ci pour continuer la procédure chirurgicale de manière sûre et en évitant la fuite du pneumopéritoine; ce kit (100) comprend:
a) un dispositif (10) de mobilisation de type pistolet qui comprend un manche (12) ergonomique qui sert d'instrument pour attraper, une première tige (11) fixée par une extrémité du manche et qui s'étend en direction essentiellement perpendiculaire par rapport au manche, une deuxième tige (14) qui peut glisser, essentiellement parallèle à la première tige, une poignée (15) près du manche, qui est attachée à la deuxième tige (14) afin de pouvoir bouger avec celle-ci, où la poignée (15) a deux extensions qui se confrontent entre lesquelles s'étend la première tige et dans lesquelles le chirurgien met les doigts pour retirer ladite poignée (15) vers le manche (12), et un cylindre ou tambour (50) de fixation auquel se connecte le bout distal de la première et deuxième tiges - ce cylindre (50) de fixation est adapté pour bouger de manière pivotante à partir d'une position de fonctionnement horizontal vers une deuxième position de fonctionnement pour atteindre un mouvement giratoire vers le haut d'au moins 90°;
b) un système de fixation (20,30,40) de l'utérus qui comprend: une patte (20) qui fonctionne de manière libre avec le cylindre de fixation (50) - cette patte (20) a une partie filetée aux bords pointus dont le diamètre diminue vers l'avant pour se fixer au col de l'utérus et termine devant par une pointe filetée (26); un extenseur (40) avec un conduit longitudinal fileté de l'intérieur pour fonctionner avec la pointe (26) de la patte (20) - cet extenseur (40) s'étend jusqu'au fond de l'utérus; et un capuchon (30) qui fonctionne avec la patte (20) pour l'aider à se fixer et à manipuler le col de l'utérus ainsi qu'à éviter la fuite du pneumopéritoine; ce kit se **caractérise par le fait qu'**il comprend également :
c) un ensemble applicateur (60) pour positionner correctement la patte (20) et le capuchon (30) dans le col de l'utérus, et qui comprend une barre avec d'un côté un système pour fonctionner librement avec la patte (20) et de l'autre côté un élément (64) pour faciliter le vissage de la patte (20) dans le col de l'utérus, et un tube coulissant (66) qui s'insère dans la barre (62), derrière le capuchon (30), pour le faire coulisser de manière uniforme le long d'une fente (61) longitudinale qui se trouve dans la barre jusqu'à une position dans laquelle il s'assemble dans la patte pour fixer le col de l'utérus.

2. Le kit de la revendication 1, pour lequel le cylindre de fixation (50) inclut en plus des moyens (57) pour retenir et libérer la patte.

3. Le kit de la revendication 1, pour lequel la patte (20) inclut en plus des moyens (24, 28) pour se fixer au cylindre de fixation.

4. Le kit de la revendication 1, pour lequel la patte (20) inclut in plus des moyens (21) pour fonctionner avec le capuchon (30).

5. Le kit de la revendication 1, pour lequel le capuchon (30) inclut des moyens (38) pour fonctionner avec la patte (20) et pour coulisser le long de la barre de l'applicateur (62).

6. Le kit de la revendication 1, pour lequel la connexion du bout distal de la première et de la deuxième tige (11, 14) du dispositif de pistolet (10) au cylindre de fixation (50) est une connexion charnière dans les deux cas, de ce fait ce cylindre de fixation peut tourner vers le haut de manière déterminée.

7. Le kit de la revendication 1, pour lequel les moyens pour retenir et libérer la patte (20) du cylindre de fixation (50) consistent en un mécanisme (22) de boulon et balle qui s'insère dans des fentes (27) et un dispositif de cale avec ressort (54, 55, 56, 57).

8. Le kit de la revendication 1, pour lequel la connexion du bout distal de la deuxième tige (14) du dispositif de pistolet (10) avec le cylindre de fixation (50) reste positionnée plus en avant que la connexion de la première tige (11) afin que le cylindre puisse tourner vers le haut quand la poignée est retirée.

9. Le kit de la revendication 1, pour lequel le dispositif de mobilisation (10) inclut des ressorts (17a, 17b) qui entourent la tige coulissante (14) pour produire le mouvement de traction et rétraction de cette tige (14) entre les positions de fonctionnement.

10. Le kit de la revendication 1, pour lequel l'extenseur (40) a une pointe (44) coupante utilisée quand il est nécessaire de traverser des tumeurs dans l'utérus.

11. Le kit de la revendication 1, pour lequel le dispositif de mobilisation (10) inclut des moyens (80) pour maintenir le système de fixation dans l'utérus à une position déterminée.
